# EUROPEAN PATENT APPLICATION

(11) **EP 4 759 272 A2**
(43) Date of publication of application: **17.06.2026**
(21) Application number: 25221523.1
(22) Date of filing: 08.12.2025
(51) Int. Cl.: A61F 2/46

(54) **RIGHT ANGLE POLY DISTRACTION TOOL**

(30) Priority: 13.12.2024 US 202463733713 P; 02.12.2025 US 202519405535
(71) Applicant: Wright Medical Technology, Inc., Memphis, TN 38117 (US)
(72) Inventor: COOK, Daniel August, Memphis, 38112 (US); DAWSON, John David, Frisco, 75034 (US)
(74) Representative: Zimmermann & Partner Patentanwälte mbB

(57) **Abstract**

A hand tool, method, and system for distracting an articulating insert from a tibia tray are disclosed.

## Description

### CROSS-REFERENCE TO RELATED APPLICATIONS

This application claims benefit to U.S. Provisional Application No. 63/733,713, filed December 13, 2024, entitled "Right Angle Poly Distraction Tool," the disclosure of which is incorporated herein by reference in its entirety.

### FIELD OF DISCLOSURE

The disclosed instruments, systems, and methods relate generally to the field of orthopedic surgical instruments.

### BACKGROUND

Many total ankle replacement prosthesis systems include an assembly of a tibia tray and a bearing component. Generally, tibia tray is first installed on to the distal end of the patient's tibia that has been prepared to receive the tibia tray. Then the bearing component is removably engaged to the bottom or distal surface of the tibia tray. It may become necessary to remove the bearing component, and a means for distracting the removable bearing component without damaging or removing the tibia tray is required.

### SUMMARY

Disclosed is a surgical hand tool to be used for dis-engaging a polymer articulating insert from a tibia tray by breaking a snap-fit engagement between the tibia tray and the polymer insert. The hand tool comprises: a first arm having a first end and a second end and a second arm having a first end and a second end. The first arm and the second arm are pivotally connected at a point between their respective first and second ends. The second end of the first arm has one or more projections that are configured to engage a corresponding recess among one or more recesses in a tibia tray. The second end of the second arm has a slot that is configured to engage a bone removal screw.

Also disclosed is a method for dis-engaging a polymer articulating insert from a tibia tray. A method includes coupling a first arm of a hand tool to a tibia tray by inserting one or more projections into one or more corresponding recesses in the tibia tray, coupling a second arm of a hand tool to a bone removal screw by engaging a slot on the second end of the second arm with the bone removal screw, and operating the hand tool to separate the bone removal screw from the tibia tray.

Also disclosed is a system for dis-engaging a polymer articulating insert from a tibia tray, a system includes a hand tool, a bone removal screw, and a tibia tray having an articulating insert. The hand tool is configured to engage with the tibia tray and bone removal screw, and operation of the hand tool separates the bone removal screw from the tibia tray.

### BRIEF DESCRIPTION OF THE DRAWINGS

These and other features and advantages of the disclosed systems and methods will be more fully disclosed in, or rendered obvious by, the following detailed description of the preferred embodiment of the disclosed systems and methods, which are to be considered together with the accompanying drawings wherein like numbers refer to like parts and further wherein:
FIGS 1-3 are side views of a surgical hand tool.
FIG. 4A-4B are side views of a second handle of a surgical hand tool.
FIG. 4C-4D are side views of a first handle of surgical hand tool.
FIG. 5 is a view of surgical hand tool.
FIG. 6 is a view of a surgical hand tool engaging a tibia tray and a bone removal screw.
FIG. 7-8 are side views of a surgical hand tool engaging a tibia tray and a bone removal screw.
FIG. 9 illustrates a close-up view of a surgical hand tool engaging a tibia tray and a bone removal screw.
FIG. 10 illustrates a close-up view of a surgical hand tool in an open position while engaging a tibia tray and a bone removal screw.
FIG. 11 illustrates a side view of a surgical hand tool engaging a tibia tray and a bone removal screw.
FIG. 12 illustrates a side view of a surgical hand tool engaging a tibia tray and a bone removal screw.
FIG. 13 illustrates a top view of a surgical hand tool engaging a tibia tray and a bone removal screw.
FIG. 14 illustrates a bottom view of a surgical hand tool engaging a tibia tray and a bone removal screw.
FIG. 15 illustrates a close-up, half section view of a surgical hand tool engaging a tibia tray and a bone removal screw.
FIG. 16 illustrates a tibia tray.
FIG. 17 illustrates a top view of a tibia tray.
FIG. 18 illustrates a half section view of a tibia tray.
FIG. 19 illustrates a rear view of tibia tray.
FIG. 20 illustrates a front view of tibia tray.
FIG. 21 illustrates a bone removal screw.
FIG. 22 illustrates a side view a bone removal screw.
FIG. 23 illustrates a surgical hand tool engaging a tray removal screw and a bone removal screw.
FIG. 24 illustrates a tibia tray.
FIG. 25 illustrates a front view of a tibia tray.
FIG. 26 illustrates a tray removal screw.

### DETAILED DESCRIPTION

This description of embodiments is intended to be read in connection with the accompanying drawings, which are to be considered part of the entire written description of the disclosed systems and methods. The drawing figures are not necessarily to scale and certain features of the systems and methods may be shown exaggerated in scale or in somewhat schematic form in the interest of clarity and conciseness. In the description, relative terms such as "horizontal," "vertical," "up," "down," "top" and "bottom" as well as derivatives thereof (e.g., "horizontally," "downwardly," "upwardly," etc.) should be construed to refer to the orientation as then described or as shown in the drawing figure under discussion. These relative terms are for convenience of description and normally are not intended to require a particular orientation. Terms including "inwardly" versus "outwardly," "longitudinal" versus "lateral" and the like are to be interpreted relative to one another or relative to an axis of elongation, or an axis or center of rotation, as appropriate. Terms concerning attachments, coupling and the like, such as "coupled," "connected" and "interconnected," refer to a relationship wherein structures are secured or attached to one another either directly or indirectly through intervening structures, as well as both movable or rigid attachments or relationships, unless expressly described otherwise. The term "operatively connected" is such an attachment, coupling or connection that allows the pertinent structures to operate as intended by virtue of that relationship. In the claims, means-plus-function clauses, if used, are intended to cover the structures described, suggested, or rendered obvious by the written description or drawings for performing the recited function, including not only structural equivalents but also equivalent structures.

The disclosed surgical tool, systems, and methods provide the ability to perform surgical procedures beyond the initial installation of an orthopedic prosthesis. In some embodiments, the disclosed systems and methods are used in performing revision surgeries. For example, the disclosed systems and methods utilize one or more foreign objects in patient's body, such as a previously installed implant (including, for example, a tibia tray) and/or other object not native to the patient's body, and surgical tools having a surface that conforms to and/or is configured to engage with a surface of the one or more foreign objects. These disclosed systems and methods advantageously improve the ability to remove an articulating insert when performing a revision or other orthopedic procedures.

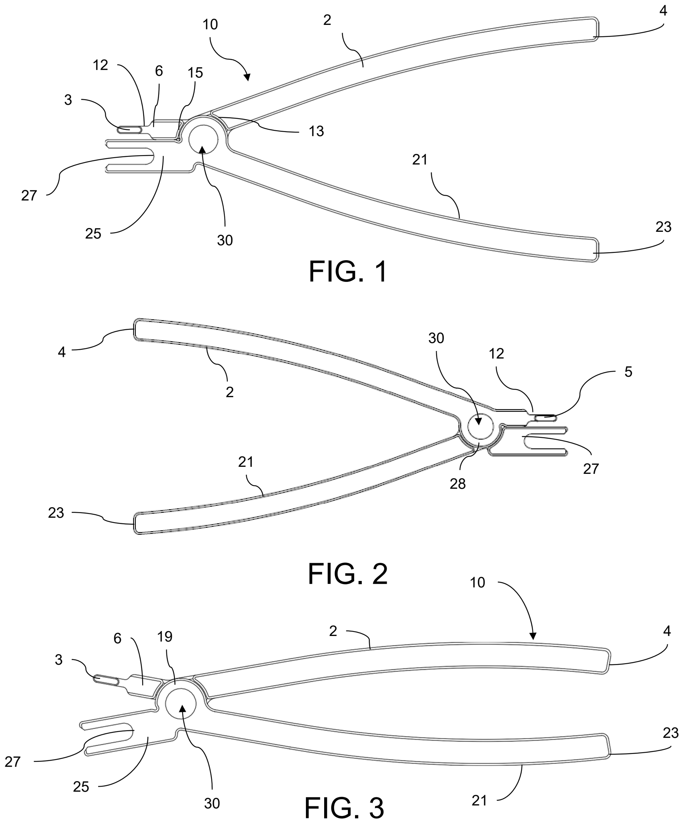

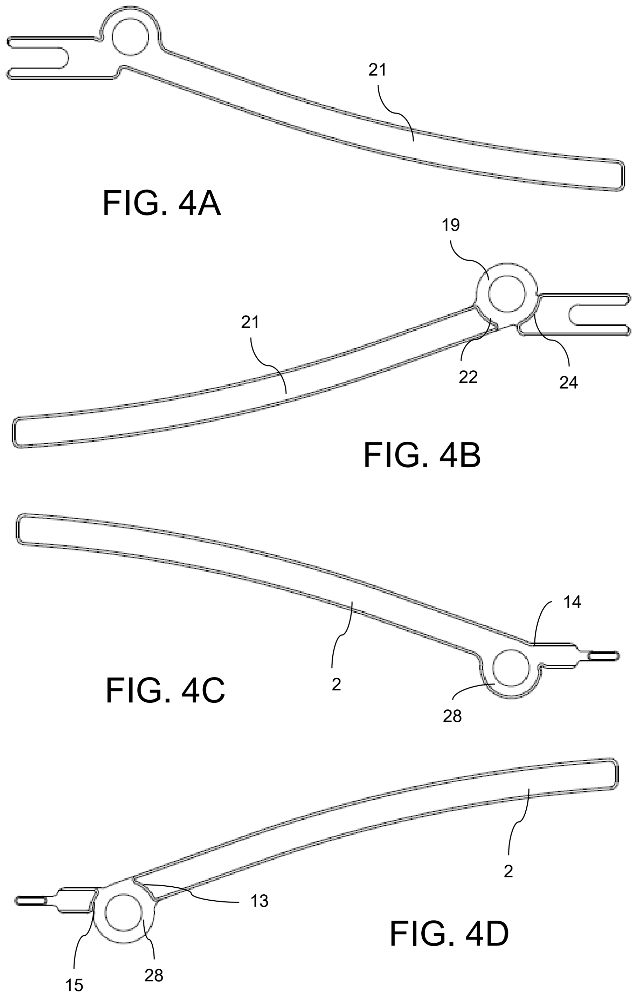

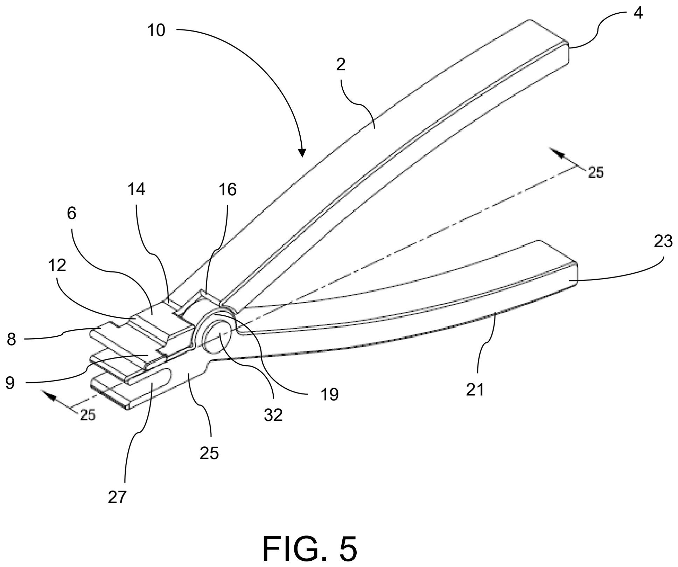

FIGS 1-5 show a first handle 2 formed with an elongated arm including a first end 4 and a second end 6. First end 4 of first handle 2 extends along a portion of first handle 2 has a slight downward curve with respect to a lateral view (for example, shown in FIG. 1). Referring to FIG. 5, second end 6 of first handle 2 has projections 8 that extend from both lateral sides of second end 6 of first handle 2. Projections 8 include elongated tabs 3 and 5 (shown in FIGS 1 and 2) that project from both lateral sides of second end 6 of first handle 2. Referring again to FIG. 1, second end 6 starts with a first diameter and tapers into a second, lesser diameter from which projections 8 project, thereby creating groove 12. First end 4 of first handle 2 and second end 6 of first handle 2 are coupled together at a portion on first handle 2, and create crease 14 (shown in FIG. 5). Crease 14 extends halfway across a top side of first handle 2, where it abuts cutout 16. First handle 2 defines cutout 16, as may be seen in FIG. 5. Referring to FIG. 4D, first handle 2 defines cutout 16 such that curved edges 13 and 15 each form an arc of a circle when viewed from the side. Referring now to FIG. 4C, first handle 2 has a slider 28. Slider 28 has the shape of a half circle and is coupled with second end 6 of first handle 2 and first end 4 of first handle 2, below crease 14. As seen in FIG. 4D, slider 28 abuts cutout 16 defined by first handle 2 and curved edges 13 and 15. Slider 28 may define a circular hole through the center or slider 28.

Still referring to FIGS. 1-5, second handle 21 is formed with an elongated arm including a first end 23 and a second end 25. First end 23 of second handle 21 extends along a portion of second handle 21 and has a having a slight upward curve with respect to a lateral view (for example, shown in FIG. 1). Referring to FIG. 1, second end 25 of second handle 21 defines cavity 27. Referring to FIG. 3, second arm 21 is formed with a slider 19. Slider 19 has the shape of a half circle and is coupled with second end 25 of second handle 21 and first end 23 of second handle 21. Referring to FIG. 5, slider 19 extends halfway across a top side of second handle 21. Slider 19 may define a circular hole through the center of slider 19.

Referring again to FIGS 1-5, pivot joint 30 couples first handle 2 and second handle 21 at a point between their respective first ends, 4 and 23, and their respective second ends, 6 and 25. As seen in FIG. 5, slider 19 of second handle 21 fits within cutout 16 defined by first handle 2 such that first handle 2 and second handle 21 are vertically aligned. Slider 19 rests against curved edges 13 and 15, as shown in FIG. 1. The radius of slider 19 is the same as the radius of curved edges 13 and 15. Slider 19 allows curved edges 13 and 15 to slide over the surface of slider 19. Referring to FIG. 2, slider 28 of first handle 21 rests against curved edges 22 and 24. Curved edges 22 and 24 have the same radius as slider 28. The circular holes through the respective centers of sliders 19 and 28 are aligned.

Pivot point 30 couples first handle 2 and second handle 21. Pivot point 30 allows movement in one planar direction. Referring to FIG. 5, cylindrical shaft 32 projects through the circular holes in the respective centers of slider 19 and 28. The diameter of cylindrical shaft is the same diameter as circular holes in the respective centers of slider 19 and 28. Cylindrical shaft 32 allows handles 2 and 21 to slide around cylindrical shaft. Cylindrical shaft 32 may lock on one or both ends of the shaft so as to prevent movement between handles 2 and 21 in a second planar direction.

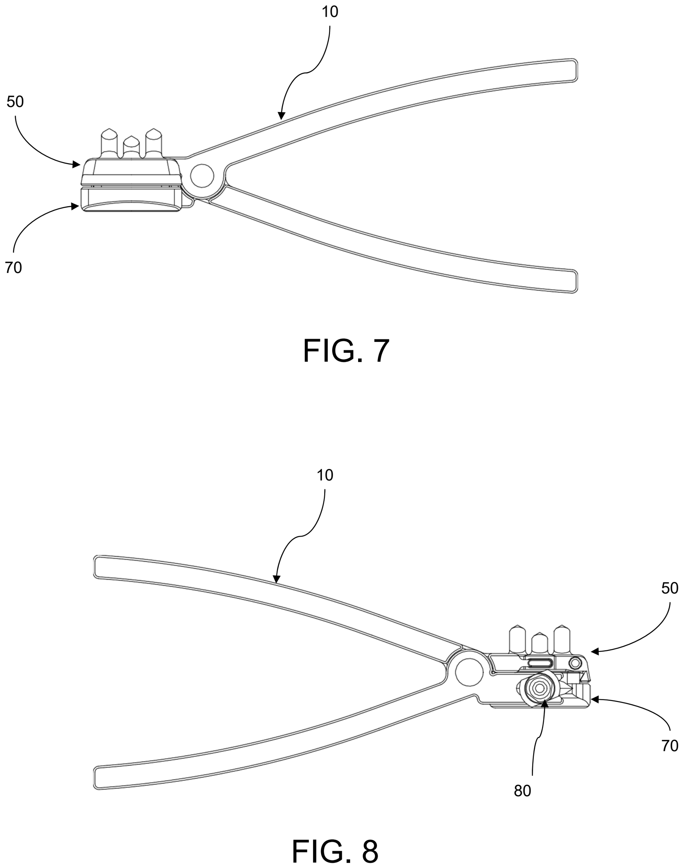

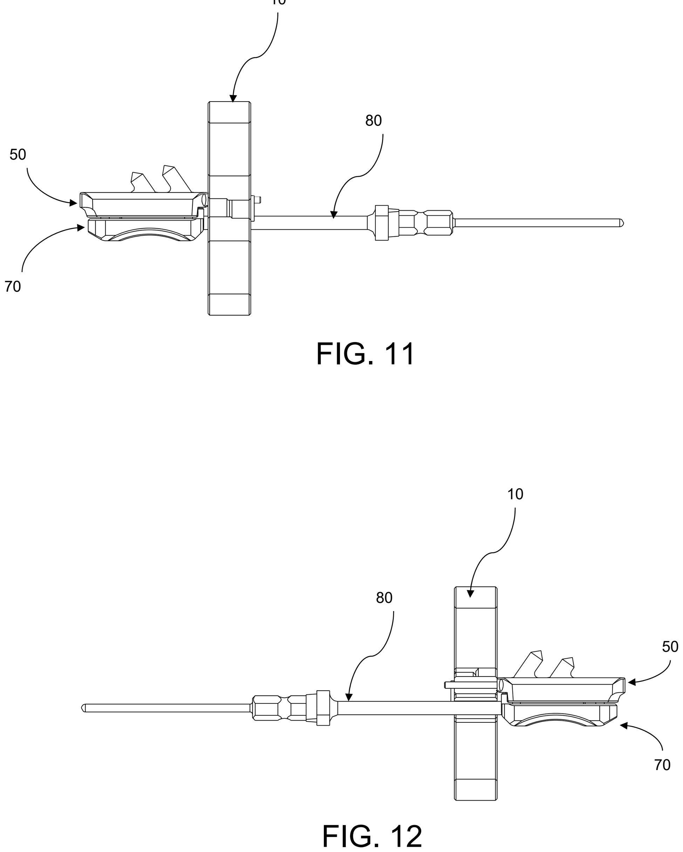

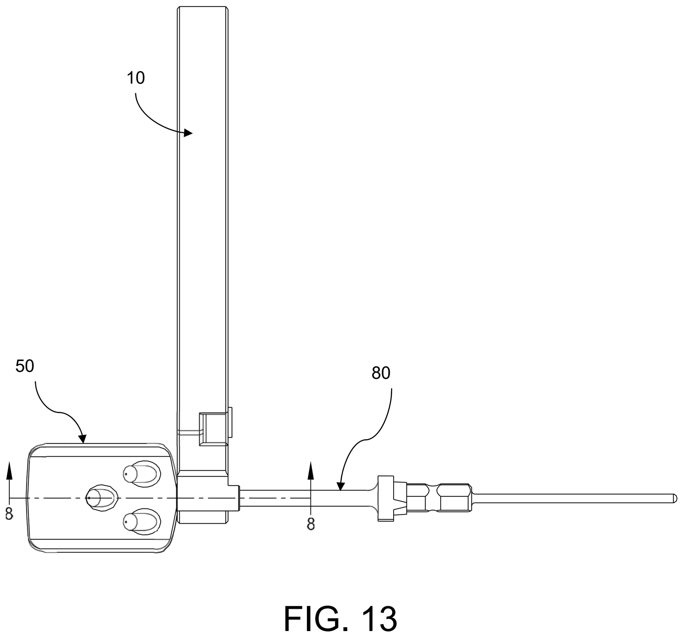

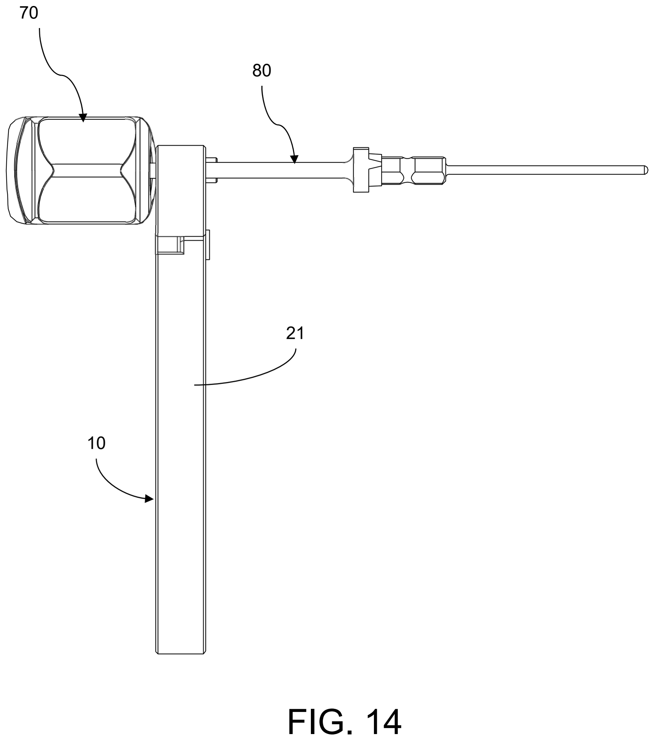

FIGS. 1-15 show hand tool 10. Hand tool 10 has first handle 2, second handle 21, and pivot joint 30. An operator may squeeze first ends 2 and 23 of hand tool 10, and sliders 19 and 28 may slide around cylindrical shaft 32 and over curved edges 13, 15 and 22, 24. First handle 2 and second handle 21 do not cross over each other, such that when an operator holds hand tool 10 by first ends 4 and 23 and squeezes first ends 4 and 23 towards each other, second ends 6 and 25 open away from each other (for example, hand tool 10 begins as shown in FIG. 1 and ends as shown in FIG. 3).

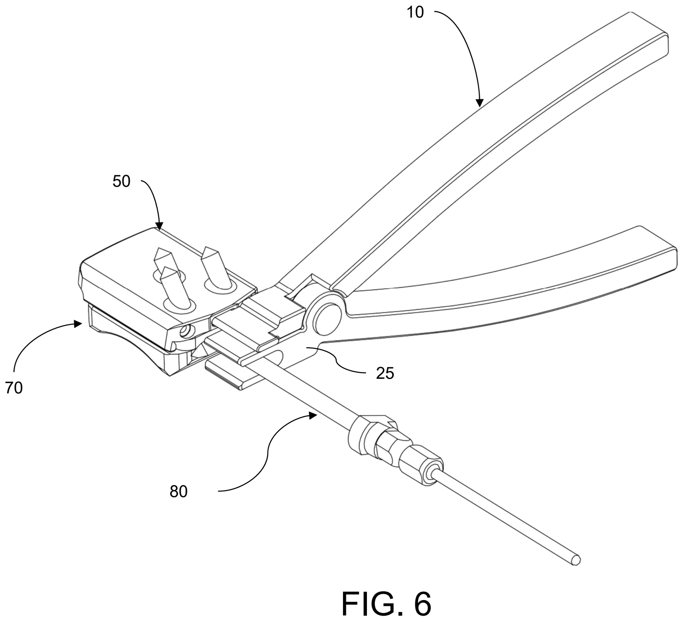

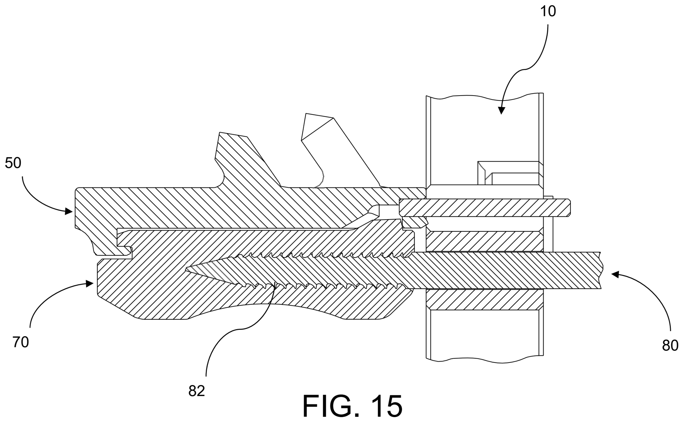

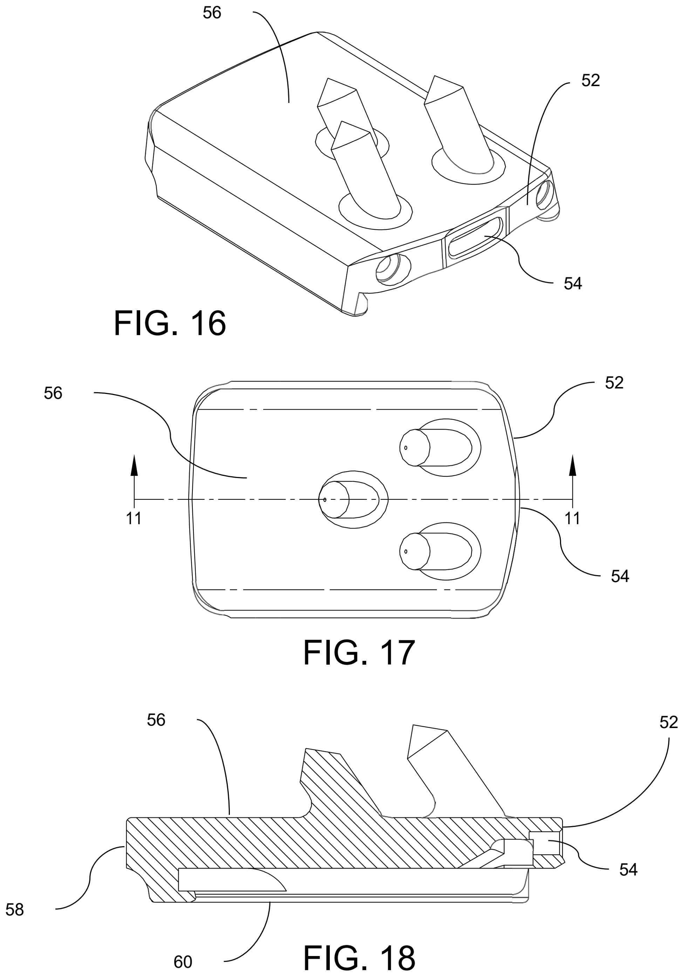

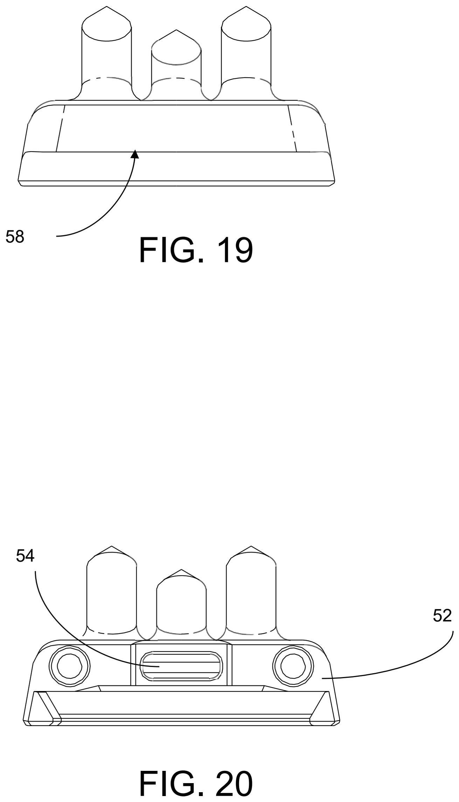

FIGS. 6-20 show tibia tray 50. Tibia tray 50 has a first end 52, a top 56, a second end 58, and a bottom 60. Referring to FIGS. 15-17 and 19, the center of first end 52 has a cutout that creates slot 54, such that first end 52 extends around slot 54, as best seen in FIGS. 15 and 17.

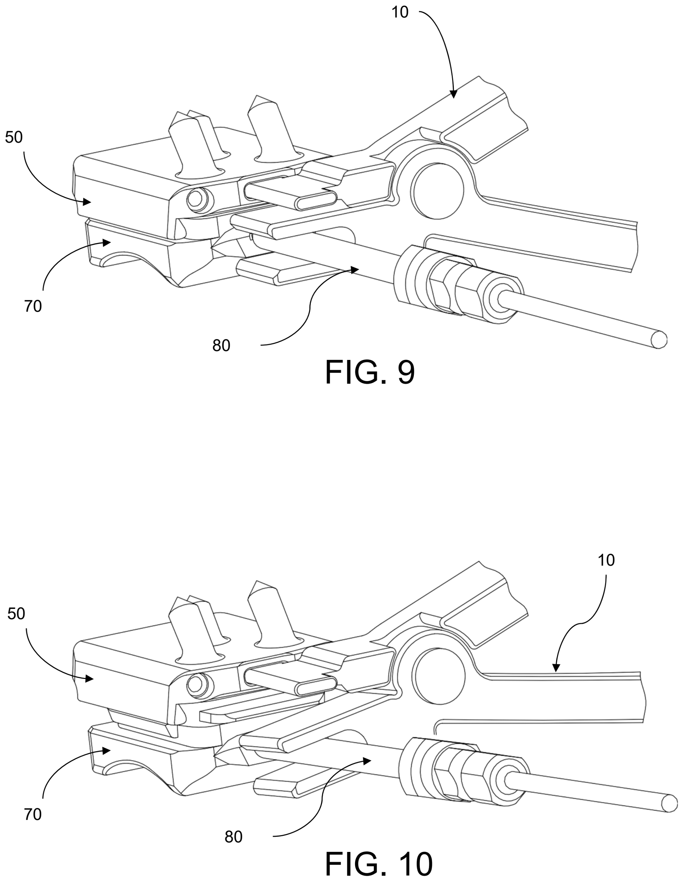

FIGS. 6-15 show articulating insert 70. Articulating insert 70 is made of a polymer material. As shown in FIGS. 6-20, articulating insert 70 may be coupled to bottom 60 of tibia tray 50 using, for example, a snap-fit engagement. As shown in FIGS. 9 and 10, articulating insert 70 may be removed from tibia tray 50.

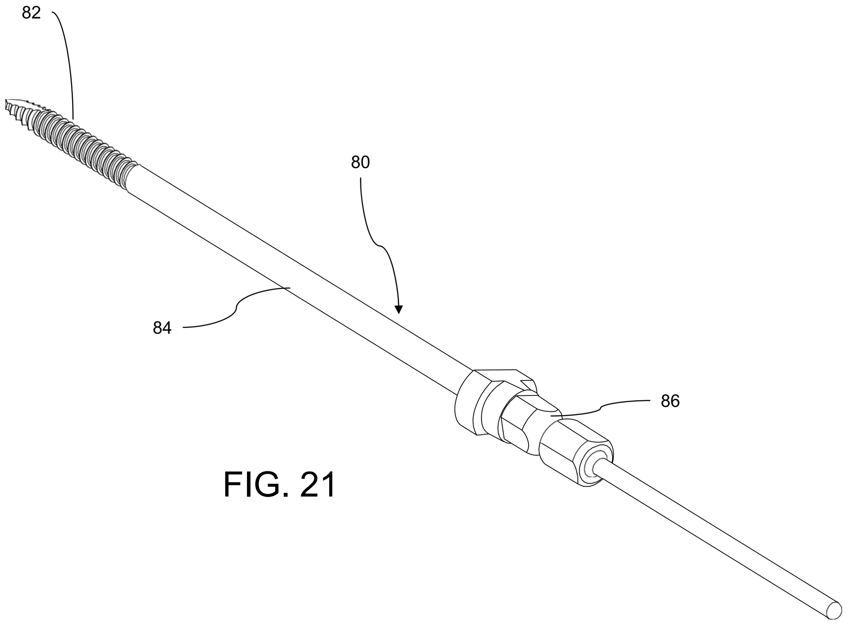

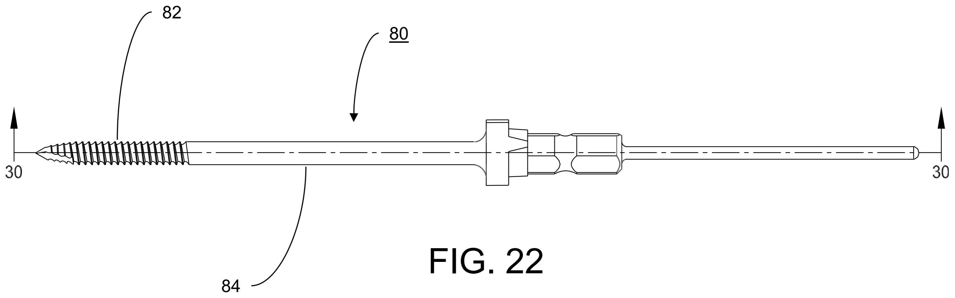

FIGS. 21 and 22 show bone removal screw 80. As seen in FIG. 22, bone removal screw 80 includes cylindrical shaft 84. Cylindrical shaft 84 of bone removal screw 80 includes threads 82. As shown in FIG. 21, bone removal screw 80 has a grip 80.

Referring to FIG. 15, bone removal screw 80 is inserted into articulating insert 14. Threads 82 of bone removal screw 80 couple bone removal screw 80 to articulating insert 70.

Referring to FIGS. 6-15, hand tool 10 is operated in with bone removal screw 80 to separate tibia tray 50 and articulating insert 70. As shown in FIG. 9, second end 25 of hand tool 10 is aligned with bone removal screw 80. Cylindrical shaft 84 of bone removal screw 80 fits within slot 27 of second end 25 of hand tool 10. As seen in FIGS. 9, 13, and 15, projection 8 elongated tab 5 of projection 8 is inserted into slot 54 of tibia tray 50. Referring to FIGS. 9-10, hand tool 10 is operated such that second end 6 of first handle 2 and second end 25 of second handle 25 open away from each other. As second end 6 of first handle 2 and second end 25 of second handle 25 open away from each other, projection 8 of second end 6 applies pressure on tibia tray 50 in one direction. Second end 25 applies pressure on bone removal screw 80 in a second direction. Bone removal screw 80 is coupled to articulating insert 70 and applies pressure on articulating insert 70. Still referring to FIG. 10, operation of hand tool 10 breaks a snap-fit connection and causes tibia tray 50 and articulating insert 70 to separate from each other.

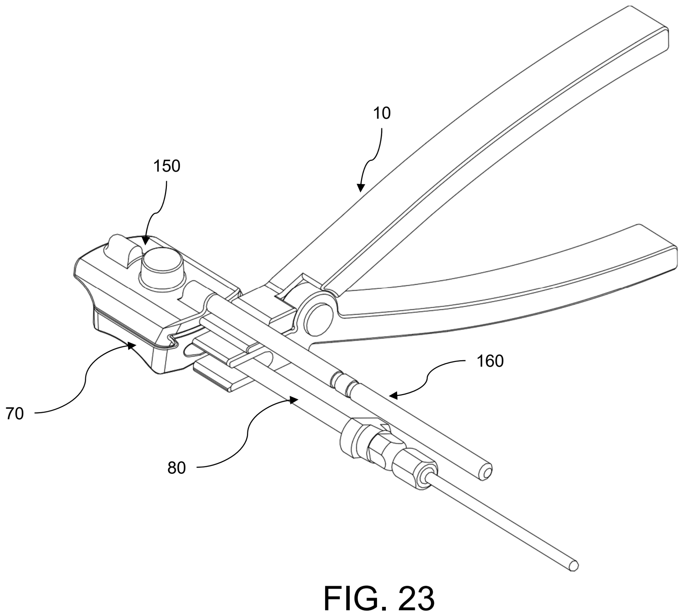

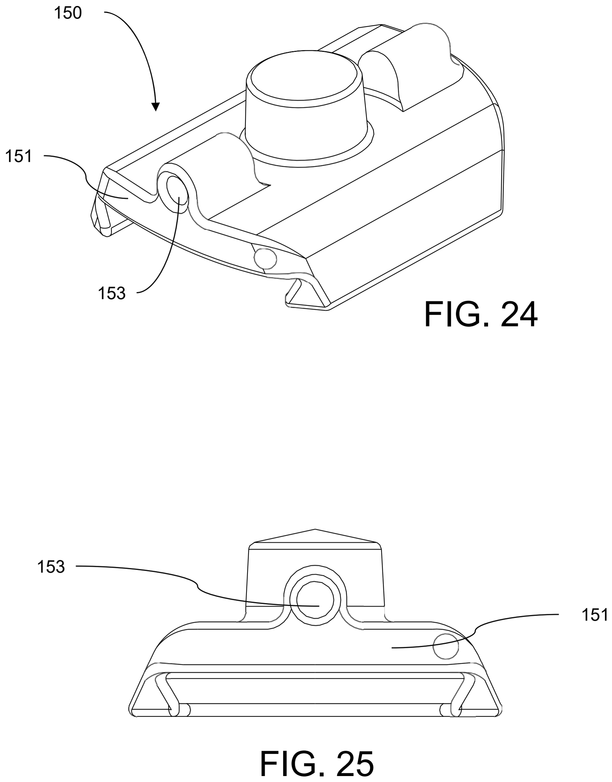

Referring now to FIGS. 24-25, tibia tray 150 has a front end 151. Front end 151 has a hollow portion 153. As shown in FIG. 23, articulating insert 70 is coupled with tibia tray 150 in a snap-fit engagement.

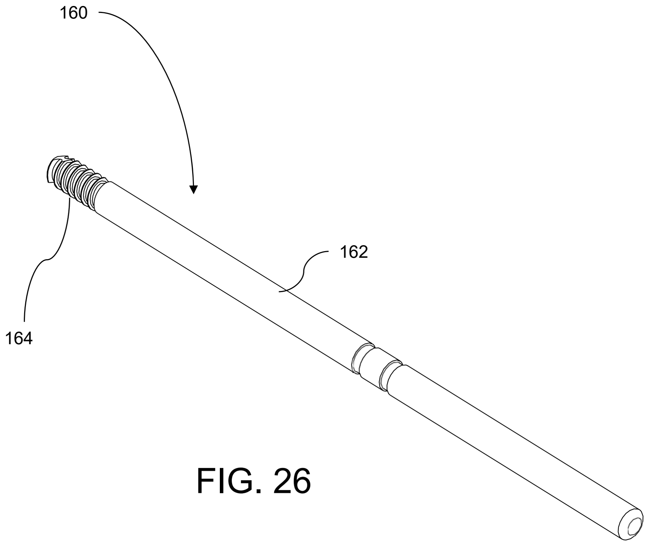

FIG. 26 shows tibia tray removal screw 160. Tibia tray removal screw 160 includes cylindrical shaft 162. Cylindrical shaft 162 of tibia tray removal screw 160 has threads 164. Referring to FIG. 23, tibia tray removal screw 160 is coupled with hollow portion 153 of tibia tray 150. Second end 25 of hand tool 10 is aligned with bone removal screw 80. Cylindrical shaft 84 of bone removal screw 80 fits within slot 27 of second end 25 of hand tool 10. Groove 12 of hand tool 10 is aligned with cylindrical shaft 162 of tibia tray removal screw 160, and cylindrical shaft 162 rests on groove 12. Hand tool 10 operates as described above and as shown in FIGS. 1 and 3. Groove 12 of second end 6 of hand tool 10 applies pressure on tibia tray removal screw 160 in one direction. Tibia tray removal screw 160 applies pressure on tibia tray 150. Second end 25 of hand tool 10 applies pressure on bone removal screw 80. Bone removal screw 80 thereby applies pressure on articulating insert 70. Operation of hand tool 10 breaks a snap-fit connection and causes tibia tray 50 and articulating insert 70 to separate from each other.
Further disclosed herein is the subject-matter of the following clauses:
1. A hand tool comprising:
   a first arm having a first end and a second end; and
   a second arm having a first end and a second end, wherein the first arm and the
      second arm are pivotally connected at a point between their respective first and second ends;
      wherein the second end of the first arm comprises one or more projections, each of the one or more projections configured to engage a corresponding recess among one or more recesses in a tibia tray; and
      wherein the second end of the second arm comprises a slot, the slot being configured to engage a bone removal screw.
2. The hand tool of clause 1, wherein the one or more projections are configured on at least one lateral side of the second end of the first arm.
3. The hand tool of clause 1, wherein the one or more projections are configured on two lateral sides of the second end of the first arm.
4. The hand tool of clause 1, wherein the first arm and the second arm do not cross over each other.
5. The hand tool of clause 1, wherein the bone removal screw is coupled to an articulating insert.
6. The hand tool of clause 5, wherein the tibia tray and articulating insert are coupled together.
7. A method comprising:
   coupling a first arm of a hand tool to a tibia tray by inserting one or more projections on a first end of the first arm into one or more corresponding recesses in the tibia tray;
   coupling a second arm of a hand tool to a bone removal screw by engaging a slot on a second end of the second arm with the bone removal screw; and
   operating the hand tool to separate the bone removal screw from the tibia tray.
8. The method of clause 7, further comprising coupling the bone removal screw to an articulating insert using a threaded connection.
9. The method of clause 8, wherein operating the hand tool separates the articulating insert and the tibia tray.
10. The method of clause 8, wherein operating the hand tool separates the articulating insert and the tibia tray by breaking a snap-fit engagement.
11. The method of clause 7, wherein the hand tool operating the hand tool at a right angle with respect to an anterior end of the tibia tray.
12. The method of clause 7, wherein operating the hand tool comprises moving a first end of the first arm towards a first end of the second arm.
13. The method of clause 12, wherein operating the hand tool comprises a second end of the first arm moving away from a second end of the second arm.
14. The method of clause 7, wherein the tibia tray is coupled to distal end of a tibia.
15. The method of clause 7, further comprising coupling a first arm of a hand tool to a tibia tray by inserting one or more projections on a lateral side of the first arm into one or more corresponding recesses in the tibia tray.
16. A system for distracting an articulating insert, comprising:
   a hand tool;
   a bone removal screw; and
   a tibia tray having an articulating insert;
      wherein the hand tool is configured to engage with the tibia tray and bone removal screw; and
      wherein operation of the hand tool separates the bone removal screw from the tibia tray.
17. The system of clause 16, wherein the bone removal screw is coupled to an articulating insert.
18. The system of clause 17, wherein operation of the surgical tool separates the articulating insert from the tibia tray.
19. The system of clause 16, wherein the hand tool is configured to operate when the hand tool is perpendicular to an anterior face of the tibia tray.
20. The system of clause 16, wherein operation of the hand tool comprises compressing a first end of a first arm towards a first end of a second arm and opening a second end of the first arm and a second end of the second arm.

## Claims

1. A hand tool comprising:
a first arm having a first end and a second end; and
a second arm having a first end and a second end, wherein the first arm and the
second arm are pivotally connected at a point between their respective first and second ends;
wherein the second end of the first arm comprises one or more projections, each of the one or more projections configured to engage a corresponding recess among one or more recesses in a tibia tray; and
wherein the second end of the second arm comprises a slot, the slot being configured to engage a bone removal screw.

2. The hand tool of claim 1, wherein the one or more projections are configured on at least one lateral side of the second end of the first arm.

3. The hand tool of any of the preceding claims, wherein the one or more projections are configured on two lateral sides of the second end of the first arm.

4. The hand tool of any of the preceding claims, wherein the first arm and the second arm do not cross over each other.

5. The hand tool of any of the preceding claims, wherein the bone removal screw is coupled to an articulating insert, wherein optionally the tibia tray and articulating insert are coupled together.

6. A method comprising:
coupling a first arm of a hand tool to a tibia tray by inserting one or more projections on a first end of the first arm into one or more corresponding recesses in the tibia tray;
coupling a second arm of a hand tool to a bone removal screw by engaging a slot on a second end of the second arm with the bone removal screw; and
operating the hand tool to separate the bone removal screw from the tibia tray.

7. The method of claim 6, further comprising coupling the bone removal screw to an articulating insert using a threaded connection.

8. The method of claim 7, wherein operating the hand tool separates the articulating insert and the tibia tray, or wherein operating the hand tool separates the articulating insert and the tibia tray by breaking a snap-fit engagement.

9. The method of any of claims 6-8, wherein operating the hand tool comprises operating the hand tool at a right angle with respect to an anterior end of the tibia tray.

10. The method of any of claims 6-9, wherein operating the hand tool comprises moving a first end of the first arm towards a first end of the second arm, wherein optionally operating the hand tool comprises a second end of the first arm moving away from a second end of the second arm.

11. The method of any of claims 6-10, wherein the tibia tray is coupled to distal end of a tibia, or the method further comprising coupling a first arm of a hand tool to a tibia tray by inserting one or more projections on a lateral side of the first arm into one or more corresponding recesses in the tibia tray.

12. A system for distracting an articulating insert, comprising:
a hand tool;
a bone removal screw; and
a tibia tray having an articulating insert;
wherein the hand tool is configured to engage with the tibia tray and bone removal screw; and
wherein operation of the hand tool separates the bone removal screw from the tibia tray.

13. The system of claim 12, wherein the bone removal screw is coupled to an articulating insert, wherein optionally operation of the surgical tool separates the articulating insert from the tibia tray.

14. The system of and of claims 12-13, wherein the hand tool is configured to operate when the hand tool is perpendicular to an anterior face of the tibia tray.

15. The system of any of claims 12-14, wherein operation of the hand tool comprises compressing a first end of a first arm towards a first end of a second arm and opening a second end of the first arm and a second end of the second arm.
